# EUROPÄISCHE PATENTANMELDUNG

(11) **EP 1 104 885 A2**
(43) Veröffentlichungstag der Anmeldung: **06.06.2001**
(21) Anmeldenummer: 00124441.7
(22) Anmeldetag: 08.11.2000
(51) Int. Cl.: G01N 27/414

(54) **Brandmelder mit Gassensoren**

(30) Priorität: 23.11.1999 DE 19956302
(71) Anmelder: SIEMENS AKTIENGESELLSCHAFT, 80333 München (DE)
(72) Erfinder: Fleischer, Maximilian, Dr., 85635 Höhenkirchen (DE); Meixner, Hans, Prof., 85540 Haar (DE); Ostrick, Bernhard, 81541 München (DE); Schwebel, Tim, 81543 München (DE)

(57) **Zusammenfassung**

Der Brandmelder enthält mindestens einen Sensor zur Gasdetektion nach dem Prinzip der Austrittsarbeitsänderung, der Leitfähigkeitsänderung oder einem anderen Transducer-Prinzip, wobei mindestens eine gassensitive Schicht vorliegt, deren Austrittsarbeit, Leitfähigkeit oder andere physikalische Meßgröße unter Exposition eines zu detektierenden Gases alleine zunimmt (abnimmt), während die Austrittsarbeit, Leitfähigkeit oder andere physikalische Meßgröße in Gegenwart eines Brandgasgemisches abnimmt (zunimmt).

## Beschreibung

Die Erfindung betrifft ein Brandmelder mit Gassensoren gemäß dem Oberbegriff des Anspruchs 1.

Aufgabe eines Brandmelders ist eine möglichst rasche und störsichere Branddetektion durch die Messung brandkorrelierter oder brandbegleitender Größen.

Zur Branderkennung und -meldung wird
1) die Hitzeentwicklung,
2) die Rauchentwicklung und
3) das Entstehen brandbegleitender Gase
herangezogen.

Bisherige Brandmelder verwenden daher zur Branddetektion einzelne oder Kombinationen folgender Detektionsverfahren:
a) Detektion der bei einem Brand entstehenden Wärme über eine Temperaturmessung,
b) Detektion des entstehenden Aerosols (Rauch) über optische Verfahren wie zum Beispiel
   ba) Streulichtmessung und
   bb) Lichtschwächung im optischen Bereich,
c) Detektion brandbegleitender Gase mit Hilfe von Brandgassensoren. Dazu gehören:
   ca) elektrochemische Sensoren (zum Beispiel für CO₂)
   cb) Infrarotsensoren (Strahlungsschwächung durch Absorption von IR-Licht im charakteristischen Spektralbereich)
   cc) halbleitende (resistive) Sensoren auf der Basis von Metalloxiden
   cd) massensensitive Sensoren (zum Beispiel Quarzdickenschwinger, Oberflächenwellenbauelemente)
   ce) Ionisationsmelder mit radioaktiver Quelle, welche das Brandaerosol über eine Messung der Ionisierbarkeit der Brandgase nachweisen.

Die einzelnen verwendeten Detektionsverfahren sind weitgehend optimiert. Sie besitzen nur noch geringes Verbesserungspotential. Auf ihnen basierende Melder weisen jedoch in machen Applikationen Nachteile auf, so dass die im Folgenden angegebenen Anforderungen an Brand(gas)sensoren, wie
1) geringer Energieverbrauch (< 1 mW), da häufig mehr als hundert solcher Meldeeinheiten über eine lange Meldeprimärleitung mit Energie versorgt werden;
2) Langzeitstabilität und Robustheit gegenüber am jeweiligen Einsatzort auf den Melder einwirkende Umgebungseinflüsse, wie zum Beispiel starke Schwankungen der Betriebstemperatur sowie der vorhandenen Luftfeuchte einschließlich möglicher Betauung, korrosiver Atmosphäre oder Staub;
3) Täuschungssicherheit von Einzelmeldern und Meldesystemen;
4) geringe Störanfälligkeit auch unter nicht vorhersehbaren Bedingungen;
5) schnelle Branddetektion;
6) Freiheit in der Anordnung der Melder, insbesondere auch dann, wenn die Melder eine größere Bauform aufweisen;
7) Lieferung von Informationen über die Art des Brandes, um eine Klassifikation des Brandes vorzunehmen und dadurch eine rasche und angepasste Brandbekämpfung zu ermöglichen;
8) geringe Kosten einzelner Sensoren und/oder von Sensorsystemen, um einen wirtschaftlichen Einsatz zu ermöglichen
nicht hinreichend erfüllt sind.

Nachteilig bei der a) Temperaturmessung ist beispielsweise, dass der Wärmetransport über die Gesetze der thermischen Konvektion erfolgt. Diese Messmethode ist daher in der Regel sehr träge, so dass ein Feuer oder Brand nicht schon in der Schwelphase, sondern erst in der Phase des offenen Brandes erkannt wird. Weiterhin ist eine Anordnung in der Regel an der Decke zwingend. Eine Klassifikation des Brandes (offenes Feuer, Kunststoffbrand, Holzfeuer oder Schwelbrand oder ähnliches) ist nicht möglich. Die Auswahl einer geeigneten Brandbekämpfungsmethode ist dadurch erschwert.

Optische Verfahren nach b) sind im Allgemeinen gegenüber partikelhaltigen Bedingungen sehr störanfällig, da Partikel auf dem optischen System adsorbieren. Der Transport des Aerosols zum Melder geschieht wie bei a) durch thermische Konvektion und ist deshalb sehr träge und ermöglicht erst eine späte Branderkennung. Aufgrund der Rauchausbreitung müssen die Melder von nicht unerheblicher Größe an der Decke angeordnet sein. Eine Klassifikation des Brandes ist im Allgemeinen ebenfalls schwer möglich.

Die Früherkennung von Bränden durch die Detektion von Brandgasen ist ein lang untersuchtes Entwicklungsgebiet in der Brandmeldetechnik. Erste Ideen für verbesserte Eigenschaften von Brandmeldern durch Gassensoren gehen schon fast 25 Jahre zurück. Heute werden Brandgassensoren besonders als Zusatz für herkömmliche Brandmelder interessant, um zum Beispiel deren Fehlalarmsicherheit zu erhöhen.

Für eine gezielte Entwicklung von Brandgassensoren ist eine fundierte Grundlage durch die Auswertung von Gasanalysen daher schon verfügbar. Als typische Brandgase wurden neben Wasserdampf und Kohlendioxid auch Kohlenmonoxid, Stickstoffdioxid, Stickstoffmonoxid, Wasserstoff und diverse kurzkettige Kohlenwasserstoffe identifiziert. Zu letzterer Kategorie gehören unverbrannte Pyrolysefragmente (wie zum Beispiel Amide) oder ungesättigte flüchtige Kohlenwasserstoffe. Die Detektion von Brandgasen liefert also wichtige Informationen über das Auftreten und die Charakteristik eines Brandes.

Trotz dieser inhärenten Vorteile gegenüber den herkömmlichen Verfahren wie a) Temperaturmessung und Messung der b) Rauchgasentwicklung, sind auch diese Verfahren nicht frei von Nachteilen.

Bei ca) elektrochemischen Sensoren ist derzeit die Lebensdauer auf ein bis zwei Jahre begrenzt. Weiterhin sind diese Sensoren empfindlich gegenüber tiefen Temperaturen sowie geringer Feuchtigkeit.

Die cb) Infrarotsensoren zeigen ähnliche negative Eigenschaften wie die unter b) genannten optischen Verfahren zur Rauchgasdetektion.

Der Einsatz von cc) halbleitenden (resistiven) Sensoren auf der Basis von Metalloxiden ist derzeit auf spezielle Anwendungsfälle optimiert. Ein universeller Einsatz ist derzeit noch nicht möglich.

Die cd) massensensitiven Detektoren, wie Quarzdickenschwinger oder Oberflächenwellenbauelemente, weisen einen zu hohen Preis auf, wenn die Kosten für die Primärelektronik mitberücksichtigt werden.

Die ce) Ionisationsmelder mit radioaktiver Quelle werden aus Entsorgungsgründen von Herstellern und Kunden wenig akzeptiert und sind für eine Brandklassifikation nur bedingt geeignet.

Die bekannten Verfahren, von denen die Erfindung ausgeht, sind aus dem Stand der Technik in einer Vielzahl von Abwandlungen bekannt, ein wesentliches Problem dieser bekannten Verfahren ist die Auswahl geeigneter Sensorprinzipien und die Auswahl einer geeigneten Kombination von gassensitiven Schichten, um die Vielfalt der unterschiedlichen Brandszenarien (offenes Feuer, Schwelbrand, Kunststoffbrand, Holzbrand etc.) abzudecken.

Aus dem Stand der Technik sind nun verschiedene Maßnahmen bekannt, um die geschilderte Problematik zu entschärfen. Es ist bekannt, die Leistungsfähigkeit der Melder durch die Kombination mehrerer Detetionsprinzipien zu verbessern. Solche Melder sind unter dem Stichwort "Kombimelder" bekannt. Durch eine geeignete Auswahl entsprechender Sensorprinzipien lässt sich eine Vielfalt unterschiedlicher Brandszenarien abdecken.

Gemäß dem Stand der Technik ist es derzeit dennoch nicht möglich, die große Vielfalt der unterschiedlichen Brandszenarien mit einem einzelnen Brandmeldesystem abzudecken. Man verzichtet daher bewußt auf eine Klassifikation des Brandes.

Ein Beispiel für die Anwendung von Gassensoren zur Branderkennung ist ein an der Justus-Liebig-Universität, Gießen, entwickeltes Sensorarray zur "Schwelbranderkennung" in Braunkohlekraftwerken, wie es in D. Kohl "Detection of smouldering fire by gas emission" in Proceedings AUBE95, Duisburg, 1995 pp 223-239 beschrieben wird. Hier werden drei Sensoren auf der Basis von Zinndioxid (SnO₂) verwendet, die Kohlenmonoxid (CO) Wasserstoff (H₂) und Stickoxide (NOₓ) erkennen. Zur optimalen Funktion sind die Signaturen dieser Gase bzw. Sensorantworten in einem EPROM abgelegt und werden mit den gemessenen Werten verglichen. Einer dieser Sensoren, dessen Leitfähigkeit bei Anwesenheit von NO absinkt, hat zusätzlich eine Kontrollfunktion, um Fehlalarme durch Störgase zu eliminieren. Steigt die Leitfähigkeit dieses Sensors, so wird das Signal der beiden anderen nicht berücksichtigt. Die Sensoren in diesem System sind genau auf die Anwendung im Kohlekraftwerk abgestimmt. Diese Spezialisierung in Kombination mit der hohen aufzubringenden Heizleistung für die drei Sensoren verhindert die Anwendung als universellen Brandmelder.

Ein weiteres Sensorsystem zur Branderkennung, beschrieben bei A. Hensel "Multigassensoren auf optoelektronischer Basis und deren Einsatz in der Branderkennung" in Proceedings AUBE99, 1999, Duisburg pp. 92-104 beruht auf einem optischen Sensorsystem, in dem mindestens zwei organische Indikatorfarbstoffe ihre Färbung unter basischen und sauren Gasen ändern. Diese liegen im optischen Weg einer Leuchtdiode, so dass die Lichtintensität nach der durchstrahlten Strecke zur Branderkennung ausgewertet werden muß. Ein solches System ist zwar universell einsetzbar, es fehlt allerdings an der notwendigen Selektivität, um eine Brandklassifikation vorzunehmen. Des weiteren ist in diesem System die Stabilität der Schichten noch nicht nachgewiesen. Es ist anzunehmen, dass diese elektrochemischen Sensoren ähnlich wie herkömmliche elektrochemische Sensoren eine kurze Lebensdauer aufweisen.

Dieses zweite System weist eine hohe Empfindlichkeit gegenüber Störgasen auf, da der Melder bereits reagiert, wenn einzelne Gase in erhöhter Konzentration auftreten. Des weiteren sind Querempfindlichkeiten, d. h. das Ansprechen auf Gase mit ähnlichem chemischem Verhalten nicht ausgeschlossen.

Der Erfindung liegt somit die Aufgabe zugrunde, einen Brandmelder auf der Basis von Gassensoren vorzustellen, der universell einsetzbar ist, eine hohe Selektivität aufweist, unempfindlich gegen Störgase und Querempfindlichkeiten ist und der preisgünstig herstellbar ist.

Diese Aufgabe wird erfindungsgemäß gelöst durch einen Brandmelder mit Gassensoren mit den Merkmalen des Anspruchs 1.

Vorteilhafte Ausführungen und Weiterbildungen der Erfindung sind in den Unteransprüchen angegeben.

Der erfindungsgemäße Brandmelder mit Gassensoren geht davon aus, dass sich die Eigenschaften eines Materials in Gegenwart von einem Zielgas (Brandgas) auf eine bestimmte Art und Weise verändern, während sich die Eigenschaften in Gegenwart mehrerer Gase, z. B. unter Exposition mit einem Brandgasgemisch, auf eine andere Art und Weise verändert. Der wesentliche Gedanke der Erfindung besteht nun darin, dass mindestens ein Sensor zur Gasdetektion nach dem Prinzip der Austrittsarbeitsänderung, der Leitfähigkeitsänderung oder einem anderen Tranducerprinzip vorgesehen ist, der eine gassensitive Schicht aufweist, deren Austrittsarbeit, Leitfähigkeit oder andere physikalische Meßgröße unter Exposition mit einem zu detektierenden sensitiven Gas allein zunimmt (abnimmt), während die Austrittsarbeit, Leitfähigkeit oder andere physikalische Meßgröße unter Exposition mit einem Brandgasgemisch abnimmt (zunimmt).

Weiterhin geht die Erfindung davon aus, dass es gassensitive Materialien gibt, welche ihre Eigenschaften sowohl in Gegenwart eines einzelnen Gases als auch in Gegenwart mehrerer Gase, d. h. beispielsweise in Gegenwart eines Brandgasgemisches, in ähnlicher Weise verändern. Der erfindungsgemäße Brandmelder mit Gassensor sieht daher vor, dass zusätzlich oder alternativ mindestens ein Sensor zur Gasdetektion nach dem Prinzip der Austrittsarbeitsänderung, der Leitfähigkeitsänderung oder einem anderen Transducerprinzip vorhanden ist, der eine gassensitive Schicht aufweist, dessen Austrittsarbeit, Leitfähigkeit oder andere physikalische Meßgröße sowohl unter Exposition mit einem zu detektierenden Gas allein als auch unter Exposition mit einem Brandgasgemisch abnimmt oder zunimmt.

Ein solcher Brandmelder ist universell einsetzbar und durch die Wahl geeigneter Materialien kann eine hohe Selektivität als auch eine Unempfindlichkeit gegenüber Störgasen und Querempfindlichkeiten erreicht werden.

Gemäß der Erfindung sind Stoffe aus der Materialgruppe der Phthalocyanine, wie z. B. H₂Pc, CuPc, NiPc, FePc, PbPc als Komponenten für solche gassensitiven Schichten besonders geeignet. So kommen beispielsweise die Zielgasse NO und NO₂ in allen Testbränden als Brandgase vor. Zur Detektion dieser Stickoxyde sind Phthalocyanine besonders geeignet. Dabei handelt es sich um organische Halbleiter mit p-Leitfähigkeit, die schon seit ca. 15 Jahren als gassensitive Materialien untersucht werden. Diese wurden als reine Gassensoren schon zusammen mit optischem Auswählverfahren, als resistive Sensoren, als sensitive Schichten auf Schwingquarzen und in Feldeffekttransistoren ohne Luftspalt verwendet.

Die Attraktivität dieser gassensitiven Materialgruppe beruht auf der Tatsache, dass es in nahezu allen Ausleseverfahren eine hohe Sensitivität auf oxidierende Gase wie Chlor und Stickstoffdioxyd bereits im ppb-Bereich zeigt.

In einer weiteren Ausgestaltung der Erfindung ist vorgesehen, die Gassensoren auf der Basis von Phthalocyaninen in regelmäßigen Abständen mit höherer Temperatur zu beaufschlagen. Diese Beaufschlagung mit erhöhtem thermischem Eintrag bewirkt eine Rückreaktion bzw. die Desorption adsorbierter Moleküle durch die Exposition der sensitiven Schicht mit Luft. Hierdurch erfolgt eine Regeneration des Sensors. Eine Temperatur von etwa 150°C soll hierbei nicht überschritten werden, da das Material ab ca. 200°C zu sublimieren beginnt.

Bei den resistiven Sensorprinzipien und in den Sensoren, die auf der Änderung der Austrittsarbeit beruhen, zeigen die Phthalocyanine H₂Pc, CuPc, NiPc, FePc, PbPc stets qualitativ das gleiche sensitive Verhalten auf Stickoxyde (NOₓ) und Gasgemische mit NOₓ, d. h. die Sensitivität hat dasselbe Vorzeichen.

In Untersuchungen zu dem Verhalten der Austrittsarbeitsänderung von Phthalocyaninschichten in verschiedenen Testbränden mit Hilfe der Kelvin-Methode wurde gefunden, dass bestimmte Phthalocyanine unter Exposition mit dem Brandgasgemisch nicht mehr das oben beschriebene Verhalten aufweisen. Diese Reaktion ist einmalig und scheint eine besondere Eigenschaft des Brandgasgemisches zu sein. Daher kann gemäß dieser Erfindung eine Kombination eines Gassensors mit dem herkömmlichen Verhalten mit einem Gassensor, welcher das eben beschriebene außergewöhnliche Verhalten zeigt, besonders gut zur Branddetektion eingesetzt werden.

Dies ist insbesondere dann der Fall, wenn Sensoren auf der Basis dieser Sensormaterialien als Sensorarray oder als Teil eines Sensorsarrays eingesetzt werden.

Diese Eigenschaft kann dann insbesondere zur Reduzierung von Falschalarmen eingesetzt werden.

Die Erfindung sieht insbesondere vor, dass diejenigen gassensitiven Schichten, deren physikalische Messgrößenänderung aufgrund einer Exposition mit einem Gas allein und aufgrund einer Exposition mit einem Brandgasgemisch dasselbe Vorzeichen aufweist, PbPc enthält.

Weiterhin sieht die Erfindung vor, dass diejenigen gassensitiven Schichten, deren physikalische Messgrößenänderung aufgrund einer Exposition mit einem Gas allein zunimmt und unter Exposition mit einem Brandgasgemisch abnimmt, CuPc enthält.

Die Kombination eines Sensors auf der Basis von z. B. PbPc und einem Sensor z. B. auf der Basis von CuPc nach dem Prinzip der Austrittsarbeitsänderung, der Leitfähigkeitsänderung oder einem anderen Transuserprinzip kann zur Reduzierung von Falschalarmen eingesetzt werden. Reagieren beispielsweise beide Sensoren in dieselbe Richtung, so liegt kein Brand vor und es wir kein Alarm gegeben bzw. in Kombination mit anderen Brandmeldern wird deren Signal zurückgesetzt. Reagieren die beiden Sensoren jedoch mit unterschiedlichen Vorzeichen, so ist dies ein Indiz für das Vorliegen eines Brandgasgemisches und es kann ein Brandalarm ausgegeben werden.

Eine solche Reaktion ist besonders unempfindlich gegenüber Störgasen und Störempfindlichkeiten, da die Phthalocyanine in der Regel bei Gasexposition im Nicht-Brandfall bekanntermaßen in dieselbe Richtung reagieren.

Die Erfindung sieht weiterhin vor, dass als Messsignal, welches zur Anzeige eines Brandes dient, direkt die Änderung der physikalischen Messgröße der gassensitiven Schicht unter Exposition mit einem sensitiven Gas oder Gasgemisch herangezogen wird. Alternativ oder zusätzlich sieht die Erfindung vor, als Messsignal zur Branddetektion eine ein- oder mehrfache zeitliche Ableitung der Änderung der physikalischen Messgröße der gassensitiven Schicht unter Exposition mit einem sensitiven Gas oder Brandgasgemisch zu verwenden. Auch in diesem Fall gilt die unterschiedliche Richtung des Signals zweier wie oben angegebener Sensoren als Brandkriterium.

Eine solche Anordnung, wie sie oben beschrieben wurde, muß nicht zum direkten Brandalarm herangezogen werden, sondern kann auch dazu eingesetzt werden, die Falschalarmsicherheit herkömmlicher Brandmelder herabzusetzen. So kann z.B. ein herkömmlicher Brandmelder mit zwei Betriebsmodi ausgerüstet werden: ein empfindlicher Modus, in dem gearbeitet wird, falls die beiden Gassensoren entgegengesetzte Signale zeigen und ein unempfindlicher, falls sie nicht reagieren oder gar gleich reagieren. Der Grenzfall davon wäre, daß bei gleicher Sensorreaktion der Gassensoren die Signale der herkömmlichen Brandmelder annuliert werden.

Eine besonders vorteilhafte Ausgestaltung der Erfindung sieht vor, daß das Meßsignal zur Branddetektion ein Differenzsignal zweier Sensoren ist, wobei der eine Sensor eine gassensitive Schicht aufweist, deren Austrittsarbeit, Leitfähigkeit oder andere physikalische Meßgröße unter Exposition mit einem zu detektierenden Gas allein zunimmt (abnimmt), während die Austrittsarbeit, Leitfähigkeit oder andere physikalische Meßgröße unter Exposition mit einem Brandgasgemisch abnimmt (zunimmt) und daß der andere Sensor eine gassensitive Schicht aufweist, deren Austrittsarbeit, Leitfähigkeit oder andere physikalische Meßgröße sowohl unter Exposition mit einem zu detektierenden Gas allein als auch unter Exposition mit einem Brandgasgemisch zunimmt (abnimmt).

In einem solchen Fall wird ein besonders hohes Differenzsignal im Brandfall erzielt; im Nicht-Brandfall jedoch, wenn beide Sensoren auf das Auftreten eines Störgases gleich reagieren, heben sich die Signale im Idealfall auf.

Die Erfindung sieht weiterhin vor, Sensoren zur Gasdetektion nach dem Prinzip der Austrittsarbeitsänderung anzusetzen, welche als Feldeffekttransistoren mit einem Sourcebereich, einem Kanalbereich, einem Drainbereich und einer suspended Gateelektrode, einzusetzen. Ein solcher Feldeffekttransistor ist beispielsweise wie folgt realisiert:

An einen Kanalbereich grenzen ein wannenförmiger Sourcebereich und ein wannenförmiger Drainbereich. Diese drei Bereiche befinden sich auf der Oberfläche eines Grundkörpers. Der Gatebereich weist eine definierte Beabstandung zum Grundkörper auf. Der dadurch definierte Spalt zwischen der Gateelektrode und dem Grundkörper ist Bestandteil einer Gateisolierung, wobei die gassensitive Schicht in diese Gateisolierung integriert ist.

In den Spalt gelangen im Brandfall über einen Gaseinlass Brandgasmoleküle. Die Brandgasmoleküle wechselwirken mit der gassensitiven Schicht. Dies geschieht beispielsweise über eine chemische Reaktion oder indem sich die Brandgasmoleküle als Adsorbate auf der gassensitiven Schicht niederschlagen.

Durch diese Wechselwirkung der Brandgasmoleküle mit der gassensitiven Schicht entsteht ein elektrisches Potential, das sich der angelegten Gatespannung additiv überlagert. Dadurch wird der Kanalbereich kapazitiv angesteuert. Die Änderung der Kanalleitfähigkeit kann beispielsweise bei festgehaltener Drain-Source-Spannung als Änderung des Drain-Stromes detektiert werden.

Eine besonders vorteilhafte Ausführung der Erfindung sieht vor, daß ein solcher Feldeffekttransistor zur Gasdetektion nicht nur eine gassensitive Schicht auffweist, sondern zwei. Es ist vorgesehen, daß die Austrittsarbeit der ersten gassensitiven Schicht ein herkömmliches Verhalten aufweist, d.h. daß die Austrittsarbeit sowohl unter Exposition mit einem zu detektierenden Gas allein als auch unter Exposition mit einem Brandgasgemisch in ähnlicher Weise zu- oder abnimmt. Die andere gassensitive Schicht zeigt dagegen das oben beschriebene außergewöhnliche Verhalten: Die Austrittsarbeit nimmt unter Exposition mit einem zu detektierenden Gas allein zu (ab) und unter Exposition mit einem Brandgasgemisch ab (zu). Als Meßgröße erhält man anordnungsbedingt automatisch das Differenzsignal der beiden Austrittsarbeitsänderungen.

Ein solcher Sensor - bestehend aus einer Kombination aus zwei Einzelsensoren, deren Differenzsignal gebildet wird, oder ein Aufbau, der zwei gassensitive Schichten enthält, wobei eine Schicht das herkömmliche Verhalten aufweist und die andere Schicht das oben beschriebene außergewöhnliche Verhalten - kann dann wie bereits beschrieben als eigenständiger Brand-oder als Kontrollsensor eingesetzt werden. Die Alarmschwelle eines solchen Sensors würde man dann höher ansetzen als die der beiden Einzelsensoren.

Ausführungsbeispiele der Erfindung sind in den Zeichnungen dargestellt und werden im folgenden näher beschrieben. Es zeigen:
- Fig. 1: eine Darstellung einer Austrittsänderung von CuPc und PbPc als Reaktion auf eine Exposition mit 100 ppb NO,
- Fig. 2: eine Darstellung einer Austrittsänderung von PbPc und CuPc als Reaktion auf einen Testbrand TF6 (Spiritusbrand),
- Fig. 3: eine Darstellung einer Austrittsänderung von PbPc und CuPc als Reaktion auf einen Testbrand TF1 (offenes Buchenfeuer),
- Fig. 4: eine Ableitung der Sensorsignale von CuPc und PbPc beim Testfeuer TF1 (offenes Buchenfeuer),
- Fig. 5: einen Feldeffekttransistor zur Gasdetetektion mit Luftspalt und PbPc als sensitive Schicht und CuPc als Referenzschicht.

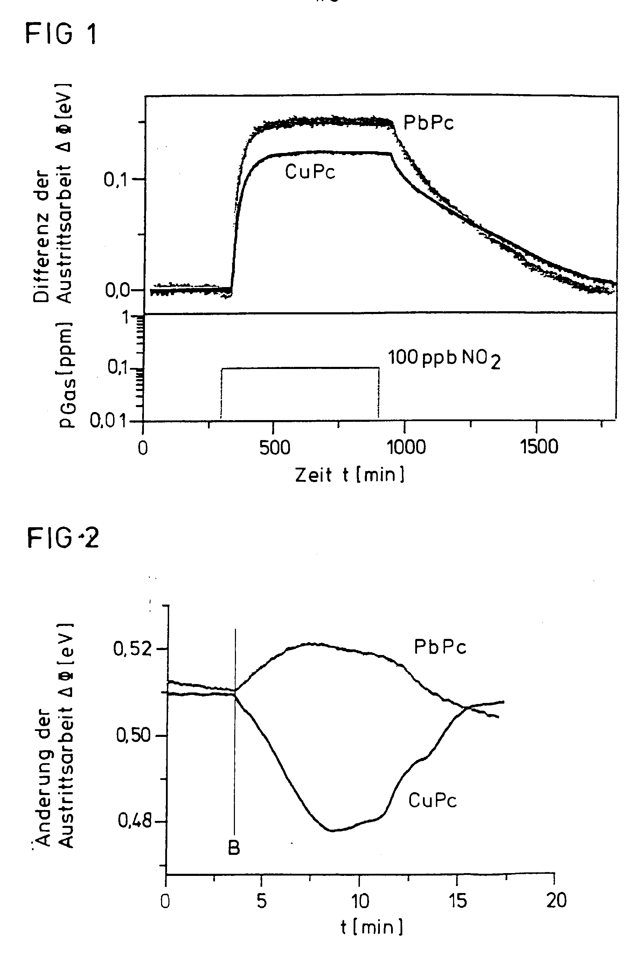

Fig. 1 zeigt die Reaktion von Kupferphthalocyanin und Bleiphthalocyanin auf eine Exposition mit 100 parts/billion Stickstoffdioxyd. Im unteren Abschnitt der Figur ist die Gasdosis über der Zeit und im oberen Bildabschnitt die entsprechende Austrittsarbeitsänderung der Kupfer- und Bleiphthalocyanine auf die Gasdosis angegeben. Der Fig. 1 ist zu entnehmen, dass nach einer Zeit t = 300 Minuten für 500 Minuten eine Gasdosis von 100 ppb NO₂ zugegeben werden. Aufgrund dieser Stickstoffdioxidzugabe erhöht sich die Austrittsarbeit von PbPc innerhalb von 100 Minuten um 150 meV. Gleichzeitig erhöht sich die Austrittsarbeit von CuPc um etwa 120 meV. In beiden Fällen ist also eine Zunahme der Austrittsarbeit unter Exposition an Stickstoffdioxyd zu beobachten.

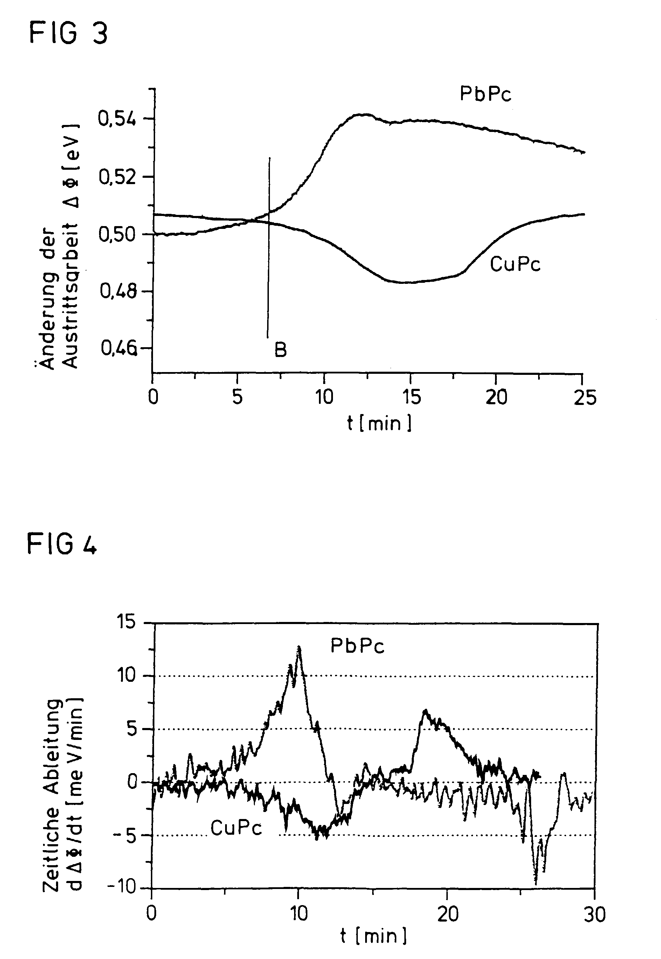

In Gegenwart eines Brandgasgemisches sind bei beiden Materialien unterschiedliche Reaktionen zu beobachten. Die Fig. 2 und 3 zeigen die Reaktionen von PbPc und CuPc im Testbrand TF6 (Spiritusbrand) bzw. im Testbrand TF1 (offenes Buchenfeuer). Die Fig. 2 zeigt die Änderungen der Austrittsarbeiten der beiden Materialien nach einem Brandbeginn B zum Zeitpunkt t = 3,5 Minuten. Aus der Figur geht hervor, dass sich die Austrittsarbeit des Bleiphthalocyanins innerhalb von drei Minuten nahezu linear um 20 meV erhöht. Demgegenüber verringert sich die Austrittsarbeit des Kupferphthalocyanins innerhalb der gleichen Zeit ebenfalls linear um 30 meV. Die Phthalocyanine CuPc und Bleiphthalocyanine PbPc weisen also eine entgegengesetzte Sensorreaktion unter Exposition mit einem Brandgasgemisch zur Branddetektion auf, während sie unter Einzeltestbedingungen in dieselbe Richtung reagieren. Eine solche Reaktion ist besonders unempfindlich gegenüber Störgasen und Querempfindlichkeiten.

Ein ähnliches Verhalten zeigt Fig. 3 bei Exposition der beiden Materialien mit dem Testbrand TF1 (offenes Buchenfeuer). Nach einem Brandbeginn B zum Zeitpunkt t = 7,5 Minuten erhöht sich die Austrittsarbeit des Bleiphthalocyanins innerhalb von etwa drei Minuten um 40 meV. In einer ähnlichen Zeitspanne reduziert sich die Austrittsarbeit des Kupferphthalocyanins um 30 meV.

Eine solche Reaktion scheint daher signifikant für ein Brandgasgemisch zu sein. Ein Sensorarray auf der Basis zweier solcher Sensoren, welche bei Exposition mit einem Brandgas unterschiedliche Reaktionen zeigen, kann daher vorteilhaft zur Branddetektion benutzt werden. Die Sensoren werden so im Array verschaltet, dass nur ein Brandalarm ausgegeben wird, falls beide in unterschiedliche Richtungen reagieren.

Figur 4 zeigt die Reaktionen der Sensorsignale von Kupferphthalocyanin und Bleiphthalocyanin beim Testfeuer TF1, wenn anstelle der Sensitivität direkt die zeitliche Ableitung dieses Signals verwendet wird. Nach Brandbeginn B (Zeitpunkt t = 6 min.) weist die zeitliche Ableitung der Austrittsarbeit wiederum bei beiden Materialien unterschiedliches Vorzeigen auf. Beide weisen eine maximale Änderungsgeschwindigkeit zum Zeitpunkt t = 10 Minuten auf, wobei der Wert d( 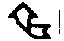 ↙)/dt = +10 meV/ min bei Bleiphthalocyanin beträgt und d( 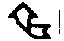 ↙)/dt = -5 meV/min bei Kupferphthalocyanin. Ein unterschiedliches Vorzeichen der zeitlichen Änderung d( 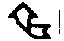 ↙)/dt der Austrittsarbeiten wird somit als Brandkriterium verwendet.

Eine Betriebsweise, in der man das Differenzsignal der beiden Sensoren betrachtet, ist ebenfalls denkbar. Ein besonders hohes Differenzsignal wird im Brandfall erzielt; im Nicht-Brandfall jedoch, wenn beide Sensoren gleich reagieren heben sich die Signale im Idealfall auf. Besonders vorteilhaft ist es, wenn die Brandgassensoren als Feldeffekttransistoren ausgebildet sind. In einem solchen Feldeffekttransistor lässt sich ein Differenzsignal sogar in einem Sensorelement verwirklichen.

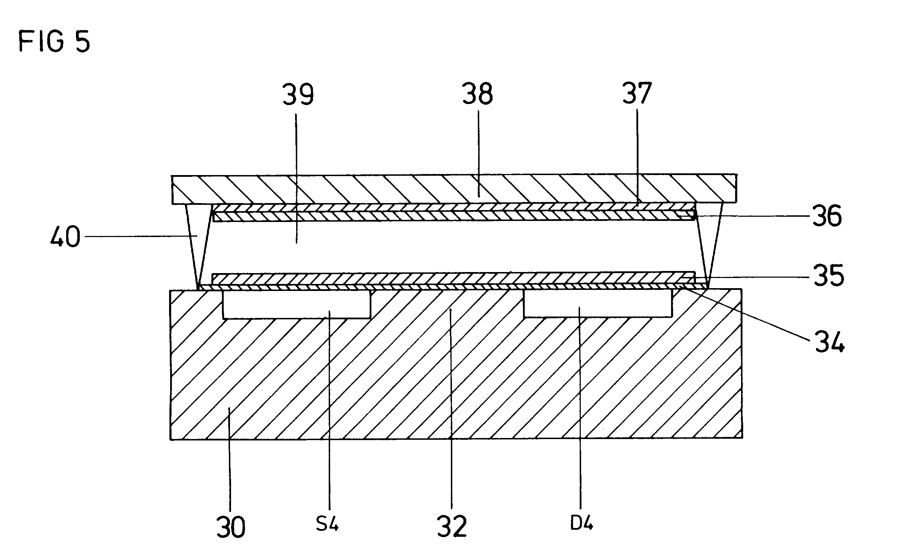

Figur 5 zeigt einen solchen Feldeffekttransistor zur Gasdetektion. Ein solcher Feldeffekttransistor ist in der Regel auf einem Siliziumsubstrat (Substratkörper) 30 angeordnet. In der Oberfläche des Substratkörpers 30 befinden sich wannenförmig ein Sourcebereich S4 und ein Drainbereich D4. Zwischen dem Sourcebereich S4 und dem Drainbereich D4 befindet sich ein Kanalbereich 32. Sourcebereich S4, Kanalbereich 32 und Drainbereich D4 sind mit einer dünnen Passivierungsschicht 34 überzogen. Die Passivierung 34 besteht beispielsweise aus Siliziumnitrit (Si₃N₄). Auf der Passivierung 34 befindet sich eine erste gassensitive Schicht 35. Diese besteht beispielsweise aus Kupferphthalocyanin (CuPc). In einem gewissen Abstand zu dieser gassensitiven Schicht 35 befindet sich eine Gate-Elektrode 38. Diese Gate-Elektrode 38 ist mit Hilfe von Abstandshaltern 40, welche auf dem Substratkörper 30 aufsitzend fixiert sind, beabstandet. Dadurch bildet sich zwischen dem Sourcebereich S4, dem Kanalbereich 32, dem Drainbereich D4 und der beabstandeten Gate-Elektrode 38 ein Luftspalt 39. Auf der dem Substratkörper 30 zugewandten Seite der Gate-Elektrode 38 befindet sich ein Gate-Metall 37. Dieses Gate-Metall 37 ist wiederum mit einer gassensitiven Schicht 36 bedeckt. Diese gassensitive Schicht 36 besteht beispielsweise aus Bleiphthalocyanin (PbPc).

Als Messgröße dieses Devices erhält man anordnungsbedingt automatisch das Differenzsignal 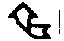 ↙_{CuPc}- 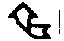 ↙_{PbPc}. Ein solcher Sensor gemäß Fig. 5 kann dann, wie bereits beschrieben, als eigenständiger Brand- oder als Kontrollsensor eingesetzt werden.

## Patentansprüche

1. Brandmelder mit Gassensoren,
**dadurch gekennzeichnet,** dass mindestens ein Sensor zur Gasdetektion nach dem Prinzip der Austrittsarbeitsänderung, der Leitfähigkeitsänderung oder einem anderen Transducerprinzip vorgesehen ist, der mindestens eine gassensitive Schicht aufweist, deren Austrittsarbeit, Leitfähigkeit oder andere physikalische Meßgröße unter Exposition mit einem zu detektierenden Gas allein zunimmt (abnimmt), während die Austrittsarbeit, Leitfähigkeit oder andere physikalische Meßgröße in Gegenwart eines Brandgasgemisches abnimmt (zunimmt) und/oder dass mindestens ein Sensor zur Gasdetektion nach dem Prinzip der Austrittsarbeitsänderung, der Leitfähigkeitsänderung oder einem anderen Transducerprinzip vorgesehen ist, der eine gassensitive Schicht aufweist, dessen Austrittsarbeit, Leitfähigkeit oder andere physikalische Meßgröße sowohl in Gegenwart eines zu detektierenden Gases allein als auch in Gegenwart eines Brandgasgemisches abnimmt oder zunimmt.

2. Brandmelder nach Anspruch 1,
**dadurch gekennzeichnet,** dass der Gassensor zur Detektion der Austrittsarbeitsänderung des gassensitiven Materiales ein Suspended Gate FET-Gassensor ist.

3. Brandmelder nach Anspruch 2,
**dadurch gekennzeichnet,** dass der Gassensor zur Detektion der Austrittsarbeitsänderung des gassensitiven Materiales ein in hybrider Aufbautechnologie erzeugter Suspended Gate FET-Gassensor ist.

4. Brandmelder nach einem der Ansprüche 1 bis 3,
**dadurch gekennzeichnet,** dass die gassensitive(n) Schicht(en) organische Farbstoffe enthalten.

5. Brandmelder nach Anspruch 4,
**dadurch gekennzeichnet,** dass die gassensitive(n) Schicht(en) Stoffe aus der Gruppe der Porphyrinfarbstoffe wie z.B. die Phthalocyanine H₂Pc,CuPc, NiPC, FePC, PbPC enthalten.

6. Brandmelder nach einem der vorangegangenen Ansprüche,
**dadurch gekennzeichnet,** dass die gassensitive(n) Schicht(en) Cobyrinsäure oder deren durch Austausch funktioneller Gruppen entstandene Derivate enthalten.

7. Brandmelder nach einem der vorangegangenen Ansprüche,
**dadurch gekennzeichnet,** dass die gassensitive(n) Schicht(en) Organische Polymere wie Polysiloxane, Polycarbonate, Polyimide oder deren durch Austausch funktioneller Gruppen entstandener Derivate enthalten.

8. Brandmelder nach einem der vorangegangenen Ansprüche,
**dadurch gekennzeichnet,** dass die gassensitive(n) Schicht(en) Anorganische Stoffe wie TiN, Carbonate wie BaCO₃, SrTiO₃, Phosphate, Oxide wie NiO, CoO, CuO enthalten.

9. Brandmelder nach einem der vorangegangenen Ansprüche,
**dadurch gekennzeichnet,** dass die gassensitive(n) Schicht(en) Edelmetalle wie Pt, Pa, Ir, Au enthalten.

10. Brandmelder nach einem der vorhergehenden Ansprüche,
**dadurch gekennzeichnet,** dass der/die gassensitive(n) Stoffe in Polymermatrizen eingebettet sind.

11. Brandmelder nach einem der vorangegangenen Ansprüche
**dadurch gekennzeichnet,** dass diejenigen gassensitiven Schichten, deren physikalische Meßgrößenänderung aufgrund einer Exposition mit einem zu detektierenden Gas allein und aufgrund einer Exposition mit einem Brandgasgemisch dasselbe Vorzeichen aufweist, PbPc enthält.

12. Brandmelder nach einem der vorangegangenen Ansprüche
**dadurch gekennzeichnet,** dass diejenigen gassensitiven Schichten, deren Austrittsarbeit, Leitfähigkeit oder andere physikalische Meßgröße aufgrund einer Exposition mit einem Gas allein zunimmt und deren Austrittsarbeit, Leitfähigkeit oder andere physikalische Meßgröße aufgrund einer Exposition mit einem Brandgasgemisch abnimmt, CuPc enthält.

13. Brandmelder nach einem der vorangegangenen Ansprüche,
**dadurch gekennzeichnet,** dass ein Meßsignal eine Änderung der Austrittsarbeit, der Leitfähigkeit oder einer anderen physikalischen Meßgröße der gassensitiven Schicht unter Exposition mit einem zu detektierenden Gas oder Brandgasgemisch ist.

14. Brandmelder nach einem der Ansprüche 1 bis 12,
**dadurch gekennzeichnet,** dass ein Meßsignal eine ein- oder mehrfache zeitliche Ableitung der Änderung der Austrittsarbeit, der Leitfähigkeit oder einer anderen physikalischen Meßgröße der gassensitiven Schicht unter Exposition mit einem zu detektierenden Gas oder Brandgasgemisch ist.

15. Brandmelder nach einem der Ansprüche 1 bis 12,
**dadurch gekennzeichnet,** dass ein Meßsignal ein Differenzsignal zweier Meßsignale zweier Sensoren zur Brandgasdetektion ist, wobei der eine Sensor eine gassensitive Schicht aufweist, deren Austrittsarbeit, Leitfähigkeit oder andere physikalische Meßgröße unter Exposition mit einem zu detektierenden Gas allein zunimmt (abnimmt), während die Austrittsarbeit, Leitfähigkeit oder andere physikalische Meßgröße in Gegenwart eines Brandgasgemisches abnimmt (zunimmt) und dass der andere Sensor eine gassensitive Schicht aufweist, deren Austrittsarbeit, Leitfähigkeit oder andere physikalische Meßgröße sowohl unter Exposition mit einem zu detektierenden Gas allein als auch unter Exposition mit einem Brandgasgemisch abnimmt oder zunimmt.

16. Brandmelder nach einem der vorangegangenen Ansprüche,
**dadurch gekennzeichnet,** dass mindestens ein Sensor zu Gasdetektion nach dem Prinzip der Austrittsarbeitsänderung ein Feldeffekttransistor ist, mit einem Sourcebereich, mit einem Kanalbereich, einem Drainbereich und einer Gateelektrode, wobei die Gatelektrode des Feldeffekttransistors zur Gasdetektion eine oder mehrere gassensitive Schichten aufweist, so dass die Kanalleitfähigkeit des Feldeffekttransistors in Abhängigkeit von einem oder mehreren zu detektierenden Gasen steuerbar ist.

17. Brandmelder nach Anspruch 16,
**dadurch gekennzeichnet,** dass mindestens ein Feldeffekttransistor eine gassensitive Schicht enthält, deren Austrittsarbeit unter Exposition mit einem zu detektierenden Gas allein zunimmt (abnimmt), während die Austrittsarbeit unter Exposition mit einem Brandgasgemisch abnimmt (zunimmt) und eine gassensitive Schicht, deren Austrittsarbeit sowohl unter Exposition mit einem zu detektierenden Gas allein als auch unter Exposition mit einem Brandgasgemisch abnimmt oder zunimmt.
